# EUROPEAN PATENT APPLICATION

(11) **EP 4 653 545 A1**
(43) Date of publication of application: **26.11.2025**
(21) Application number: 25171659.3
(22) Date of filing: 22.04.2025
(51) Int. Cl.: C12Q 1/6818, C12Q 1/6804, C12Q 1/6816

(54) **LABEL, MARKER AND METHOD FOR ANALYSING BIOLOGICAL SAMPLES**

(30) Priority: 21.05.2024 EP 24177155
(71) Applicant: Leica Microsystems CMS GmbH, 35578 Wetzlar (DE)
(72) Inventor: ALSHEIMER, Soeren, 35578 Wetzlar (DE)
(74) Representative: Schaumburg und Partner Patentanwälte mbB

(57) **Abstract**

A label (100) for analysing a biological sample is provided. The label comprises a first nucleic acid strand (102) and a second nucleic acid strand (106). The first nucleic acid strand (102) and the second nucleic acid strand (106) are configured to form at least one duplex structure. The label further comprises a first plurality of optically detectable labelling moieties (104) attached to the first nucleic acid strand (102). The label further comprises at least one binding molecule (108) configured to bind to the at least one duplex structure. In further aspects a marker (200) and a method for detecting target analytes (112, 300, 302) are provided.

## Description

### Technical field

The invention relates to a label for analysing biological samples comprising a plurality of labelling moieties and a binding molecule, as well as a corresponding marker. In a further aspect, a method for detecting target analytes is provided.

### Background

The use of markers with labels that comprise a large number of individual dyes finds frequent application in fields like molecular biology and diagnostics, offering unparalleled sensitivity and specificity in detecting biological entities and chemical processes, in particular with fluorescence microscopy. These markers enable the visualization of complex cellular structures, tracking of molecular interactions, and high-throughput assays with high resolution. In particular, labels with a large number of dyes may result in bright labels.

However, the incorporation of numerous individual dyes within markers introduces a set of challenges, most notably self-quenching. Self-quenching occurs when the proximity of dyes within a marker leads to non-radiative energy transfer among them, significantly diminishing the emission efficiency and the fluorescence intensity that can be detected. This reduction in signal not only affects the sensitivity of measurements but also complicates quantitative analysis, making it difficult to accurately determine the concentration of targets or the efficiency of binding events. Self-quenching further poses a considerable challenge in high-resolution studies.

### Summary

It is an object to provide a label and a marker that are bright and have efficient emission characteristics.

The aforementioned object is achieved by the subject-matter of the independent claims. Advantageous embodiments are defined in the dependent claims and the following description.

In a first aspect, a label for analysing a biological sample is provided. The label comprises a first nucleic acid strand and a second nucleic acid strand. The first nucleic acid strand and the second nucleic acid strand are configured to form at least one duplex structure. The label further comprises a first plurality of optically detectable labelling moieties attached to the first nucleic acid strand. The label further comprises at least one binding molecule configured to bind to the at least one duplex structure.

The label enables bright emission of the plurality of labelling moieties, in particular with native optical properties of the labelling moieties, at a high efficiency. The duplex structure of the nucleic acid strands provides a rigid or stiff backbone to attach the labelling moieties to. The rigidity or stiffness enables keeping the labelling moieties in place relative to each other and avoiding an aggregation of the labelling moieties, for example due to an attractive force between labelling moieties. Aggregation of labelling moieties may cause a change of their native optical properties such as their emission intensity and/or absorbance spectra. This makes unambiguous identification of the labelling moieties difficult or impossible.

Similarly, the at least one binding molecule of the label enables increasing the rigidity or stiffness of the nucleic acid strands of the label. In particular, by aligning or ordering the nucleic acid strands and/or the duplex structure around the binding molecule.

A measure of the degree of rigidity or stiffness of a nucleic acid, in particular a duplex structure, is persistence length (L_{P}). The persistence length is a mechanical parameter quantifying polymer rigidity: the higher the persistence length, the more rigid the polymer.

The first and second nucleic acid strands of the label may be synthetic nucleic acid strands, in particular. This means that the nucleic acid strands may be chemically synthesised, rather than be the product of a biochemical process of the biological sample to be analysed.

The first and second nucleic acid strands of the label may form part of a nucleic acid backbone of the label, for example. The first and second nucleic acid strands and/or the backbone may provide a structural framework, to which the at least one labelling moiety is attached.

For example, the first plurality of optically detectable labelling moieties may be fluorescent labelling moieties, the fluorescence of which may be optically detectable. In particular, the labelling moieties may be fluorophores such as a fluorescent protein, a fluorescent small molecule, or a quantum dot.

The optically detectable labelling moieties of the first plurality of labelling moieties may be attached to the phosphate, pentose or nucleobase of nucleotides of the first nucleic acid strand of the label, in particular. Alternatively or additionally, at least some of the first plurality of optically detectable labelling moieties may be attached to the second nucleic acid strand.

The at least one binding molecule may also be termed a nucleic acid binding molecule. For example, the binding of the binding molecule to the at least one duplex may be based on intermolecular forces such as hydrogen bonding, dipole-dipole interactions, and van der Waals forces. In particular, the at least one binding molecule is configured to non-covalently bind to the at least one duplex structure. Thus, the binding of the binding molecule to the at least one duplex may not be based on covalent bonds between the binding molecule and the duplex structure, in particular the first and/or second nucleic acid strands.

The binding of the binding molecules to the duplex structure may be non-specific or sequence specific. In the latter case, the nucleic acid strands, in particular the duplex structure, may comprise specific sequences configured to bind binding molecules.

In particular, the at least one binding molecule is not a nucleic acid and/or the binding molecule does not comprise a nucleotide. In particular, the at least one binding molecule may be configured to influence mechanical properties of the duplex structure, for example, increase a persistence length of the duplex structure.

In an embodiment of the label the first nucleic acid strand and the second nucleic acid strand are at least partially complementary. Thus, the first and second nucleic acid strands each may have at least partially complementary sequences. In particular, the complementary parts may form the at least one duplex structure. Thus, the duplex structure may be based on complementary base-pairing. The duplex structure is preferably a double stranded segment. For example, the duplex structure may be a secondary or tertiary structure, such as a double helix. In a particular example, the label may comprise several discontinuous double stranded segments that are the duplex structure. In particular, at least 50% of nucleotides of the first and second nucleic acid strands may be paired to each other to form the duplex structure. In order of increasing preference, the duplex structure preferably comprises at least 60%, 70%, 80% or 90% of nucleotides of the first and second nucleic acid strands. In a particular embodiment, the first and second nucleic acid strands may be parts of a single nucleic acid molecule. In this case the first and second nucleic acid strands may form a hairpin loop, when they form the at least one duplex structure. Alternatively, the first and second nucleic acid strands may be separate nucleic acid molecules.

In an embodiment of the label the first and second nucleic acid strands comprise between 20 and 600 nucleotides, in particular between 50 and 250 nucleotides. Each of the first and the second nucleic acid strands may comprise an essentially equal number of the nucleotides, in particular, such that the first and the second nucleic acid strands can hybridise to form a duplex structure.

In an embodiment of the label the first and second nucleic acid strands may comprise, in particular consist essentially of, at least one of a natural nucleic acid and a nucleic acid analogue. In particular, the first and second nucleic acid strands may consist essentially of nucleic acid analogues. This may render the nucleic acid strands resistant to degradation agents such as nucleases.

Generally, nucleic acid analogues are compounds which are structurally similar to naturally occurring RNA and DNA. Nucleic acids are chains of nucleotides, which are composed of three parts: a phosphate backbone, a pentose sugar, either ribose or deoxy-ribose, and one of four nucleobases. An analogue may have any of these altered. The nucleic acid analogue may be an artificial nucleic acid or a xeno nucleic acid. Whereas natural nucleic acids or naturally occurring nucleic acids (DNA and RNA) are generally sensitive to degradation agents such as nucleases, nucleic acid analogues are generally resistant to degradation agents such as nucleases.

In an embodiment, the at least one binding molecule is configured to intercalate with the at least one duplex structure or to bind to a groove of the at least one duplex structure. This enables an increased rigidity of the nucleic acid strands of the label. The interactions may, in particular, be with the first and/or second nucleic acid strands forming the duplex structure. When the binding molecule intercalates with the duplex structure, in particular, it may insert between stacked base pairs of the first and second nucleic acid strand duplex structure without breaking hydrogen bonds between complementary strands. This process typically involves planar, aromatic compounds that can slide between the base pairs, stabilising through π-π stacking interactions. When the binding molecule binds to a groove of the duplex structure, it may interact with the duplex of the first and second nucleic acid strand without disrupting their base pairing, instead fitting into the major or minor grooves formed by the helical structure. These grooves present chemically distinct environments due to the spatial arrangement of the sugar-phosphate backbone and base pairs, allowing specific recognition and binding by small molecules, peptides, or proteins. Groove binding often involves hydrogen bonding, van der Waals forces, and electrostatic interactions, enabling selective and reversible attachment.

In an embodiment, the label comprises a plurality of the at least one binding molecule.

In an embodiment of the label, the at least one binding molecule is configured to bind to the duplex structure transiently or permanently. This enables tuning of the optical properties of the labelling moieties, in particular over time.

The transient binding of the at least one binding molecule to the duplex structure may be a readily reversible interaction between the at least one binding molecule and the duplex structure that occurs with a high dissociation rate, in particular under physiological conditions. Thus, in this case the binding molecule may bind and unbind from the duplex structure frequently. These interactions are typically governed by weaker non-covalent forces. In this example, optical properties of the labelling moieties of the label may periodically change over time, depending on whether or not the at least one binding molecule is bound to the duplex structure. This may cause the label to appear as blinking, in particular in an optical readout.

The permanent binding of the at least one binding molecule to the duplex structure may be a high-affinity, non-covalent interactions between the at least one binding molecule and the duplex structure that occurs with a low dissociation rate, in particular under physiological conditions. Thus, in this case the binding of the binding molecule to the duplex structure may result in a highly stable and persistent complex, such that dissociation is negligible under physiological conditions. In this example, the optical properties of the labelling moieties of the label may remain essentially the same over time and appear so in an optical readout.

Examples of binding molecules include the following: ethidium bromide has a binding affinity (Kd) for DNA of approximately 0.2 µM; SYBR Green has a binding affinity (Kd) for DNA of approximately 3 nM in TE buffer, and 45 nM in TE buffer plus 100 mM NaCl; SYBR Safe, similar to SYBR Green; propidium iodide has a binding affinity (Kd) for DNA of approximately 1.5 µM; DAPI (4',6-diamidino-2-phenylindole) has a binding affinity (Kd) for DNA of approximately 300 nM; 7-AAD (7-aminoactinomycin D); Gel Red; Hoechst 33258 has a binding affinity (Kd) for DNA of approximately 1 µM; Hoechst 33342 has a binding affinity (Kd) for DNA is similar to Hoechst 33258, approximately 1 µM; Hoechst 34580 has a binding affinity (Kd) for DNA similar to other Hoechst dyes, approximately 1 µM. Generally, affinities for the cited DNA intercalating or DNA-binding dyes are in the nanomolar to micromolar range. The examples above are also examples of optically detectable binding molecules.

With respect to above transient/permanent binding definition, for example, the binding of SYBR Green to DNA with a Kd in the single nanomolar range can be considered permanent binding in relation to typical process times in biological applications such immunofluorescence or fluorescent in situ hybridization experiments. The binding affinity (Kd) for DNA of DAPI, which is in the range of 300nM, as well as Hoechst 34580, which is in the range of 1µM, can be considered to be high enough to mediate a transient binding, for example.

In an embodiment of the label the at least one binding molecule is optically detectable, e.g. by means of a light microscope, a fluorescence microscope, a confocal laser scanning microscope, or a cytometer. Examples of optically detectable binding molecules are listed above. Providing the label with optically detectable binding molecules enables providing a bright label. It may further enable providing labels with distinguishable optical properties, in particular a large number of distinguishable labels.

Alternatively, the at least one binding molecule may not be optically detectable. Examples of optically undetectable binding molecules include the following: Actinomycin D, intercalates between guanine-cytosine base pairs with a binding affinity (Kd) of approximately 1 nM in PBS; Daunorubicin intercalates into DNA with a binding affinity (Kd) of approximately 0.1 µM in PBS; Doxorubicin intercalates into DNA with a binding affinity (Kd) of approximately 0.1 µM in PBS; Mitoxantrone intercalates into DNA with a binding affinity (Kd) of approximately 0.5 µM in PBS; Proflavine intercalates into DNA with a binding affinity (Kd) of approximately 0.2 µM in PBS.

In an embodiment of the label the plurality of labelling moieties and the at least one binding molecule are configured to excitonically interact with each other, in particular for nonradiative energy transfer between them. This enables providing labels with distinguishable optical properties, in particular a large number of distinguishable labels. In particular, the labelling moieties and the binding molecules may be configured for fluorescence resonance energy transfer (FRET) and they may form FRET pairs. For example, the binding molecules may be FRET donors and the labelling moieties may be FRET acceptors.

In an embodiment, the label comprises at least 5 of the labelling moieties. This enables a bright label.

In an embodiment of the label the labelling moieties are hydrophobic labelling moieties. The label, in particular its nucleic acid strands, provides a rigid backbone for hydrophobic labelling moieties. Generally, in an aqueous solution hydrophobic labelling moieties have a tendency to aggregate. In particular, hydrophobic labelling moieties that are arranged in close proximity on a flexible single stranded DNA backbone may lead to formation of aggregates. This aggregation may change the optical properties of these labelling moieties. For example, their absorption wavelength may change and/or in particular their fluorescence brightness may decrease, compared to non-aggregated hydrophobic labelling moieties. By providing the label with the hydrophobic labelling moieties, the duplex structure with the binding molecules avoids the aggregation of the labelling moieties and therefore enables use of the hydrophobic labelling moieties at their native optical properties.

Examples of hydrophobic labelling moieties that are regularly used to analyse biological samples include ATTO 390, ATTO 425, ATTO 550, ATTO Rho12, ATTO 633, and ATTO 647N.

In contrast, hydrophilic labelling moieties may include ATTO 488, and ATTO 532, and ATTO 565 for example.

In a particular embodiment, the labelling moieties are evenly distributed along the first nucleic acid strand.

In an embodiment of the label the labelling moieties of the plurality of labelling moieties are attached to the at least one duplex structure, in particular to the part of the first nucleic acid strand that is configured to form the at least one duplex structure with the second nucleic acid strand. By attaching the labelling moieties to the duplex structure, the labelling moieties are attached to the part of the nucleic acid strands that has an increased rigidity. This enables generating labels with labelling moieties, wherein the labelling moieties may have a consistent distance from each other, that is not substantially influenced by the flexibility of the first nucleic acid strand. This avoids undesired interactions between the labelling moieties or aggregations of the labelling moieties. Alternatively, at least some of the labelling moieties may be attached to the second nucleic acid strand.

In an embodiment, the label may comprise a second plurality of labelling moieties, in particular with different optical properties to the first plurality of labelling moieties. This enables generating a large number of optically distinguishable labels.

In another aspect, a set of labels is provided comprising a plurality of labels as described herein. Each label of the set of labels comprises a plurality of labelling moieties, at least one of the plurality of labelling moieties of each label may have different optical properties compared to other labels, in particular compared to the labelling moieties of the remaining labels of the set of labels. Thus, the combination of labelling moieties of each label of the set of labels may be unique in the set of labels. This enables each label of the set of labels being optically distinguishable from the other labels of the set.

In particular, each label comprises a second nucleic acid strand and each first nucleic acid strand and second nucleic acid strand may form a duplex structure based on the same at least partially complementary sequences. Thus, each label may be generated using identical second nucleic acid strands. This enables efficiently generating the set of labels.

In case the label comprises optically detectable binding molecules, each label of the set of labels may similarly have optically detectable binding molecules with different optical properties.

The set of labels enables efficiently analysing a biological sample with a large number of target analytes, in particular distinguishing or identifying a large number of different target analytes.

In another aspect, a marker for analysing a biological sample with a plurality of target analytes is provided. The marker comprises a label as described herein and a first affinity reagent configured to specifically bind to one of the target analytes.

The label may be attached to the affinity reagent, in particular covalently or via a barcode oligonucleotide. The barcode oligonucleotide of the affinity reagent may be complementary to at least a part of either the first or second nucleic acid strands.

The affinity reagent may be an antibody, an antibody fragment, or an aptamer, in particular a nucleic acid based aptamer, for example. The affinity reagent enables specifically binding the label to a particular target analyte in the biological sample.

In an embodiment the marker comprises a first marker part comprising the first nucleic acid strand of the label and the first affinity reagent, and a second marker part comprising the second nucleic acid strand of the label and a second affinity reagent configured to specifically bind to one of the target analytes. The marker may enable to determine whether or not the target analytes that the first and second affinity reagents are configured to bind to are in close proximity. The first affinity reagent and the second affinity reagent may be configured to specifically bind to the same or different target analytes. In case the first and second affinity reagents bind to the same target analyte, the same target analyte may be detected with an increased sensitivity. In this embodiment of the marker, the labelling moieties may be hydrophobic labelling moieties, in particular. Thus, the marker may enable optically distinguishing between a first state of the marker, in which the first and second marker parts are separate from each other, and a second state of the marker, in which the first and second marker parts are in close proximity to each other such that the first and second nucleic acid strands may hybridise to each other. The hybridisation of the first marker part, in particular the first nucleic acid strand, to the second marker part, in particular the second nucleic acid strand, as well as the binding of the binding molecule to the formed duplex structure causes an increase in the persistence length of the first nucleic acid strand. This results in a reduction or avoidance of aggregation of labelling moieties. In particular, the binding molecule may be a FRET donor to the labelling moieties.

In a further aspect a set of markers may be provided comprising a plurality of markers as described herein. Each marker of the set of markers comprises one of the labels of the set of labels and an affinity reagent. Each affinity reagent is configured to specifically bind to a different target analyte. A label with unique optical properties is attached to the affinity reagent configured to specifically bind to a particular one of the target analytes.

The set of markers enables efficiently analysing a biological sample with a large number of target analytes, in particular distinguishing or identifying a large number of different target analytes.

In a further aspect, a method for detecting target analytes of a biological sample is provided. The method comprising the step of incubating the biological sample in the presence of at least one marker as described herein. The method further comprises the step of acquiring a readout of the at least one marker and the biological sample, in particular in order to determine the presence and/or location of the target analyte in the biological sample. For example, based on the readout the presence and/or location of at least one of the target analytes of the biological sample may be determined, in particular, the target analyte to which the affinity reagent of the marker is configured to specifically bind to.

The readout may be an optical readout, for example. In particular, the readout may be acquired by means of a microscope.

In particular, the biological sample may be incubated in the presence of the set of markers, each marker of the set being specific to a particular target analyte. This enables determining the identity or the presence of the respective target analytes in the biological sample.

In an embodiment of the method in the step of incubating the biological sample in the presence of the at least one marker, initially, the biological sample is incubated in the presence of (only) the first marker part, and subsequently the second marker part and the at least one binding molecule is added to the biological sample, in particular to incubate the biological sample in presence of both marker parts and the binding molecule. This enables detecting the presence of a particular target analyte with high sensitivity, or detecting whether or not two target analytes are in close proximity to each other. In particular, this embodiment makes use of the marker having the first marker part and the second marker part.

In an embodiment of the method an additional readout is acquired after incubating the biological sample in the presence of (only) the first marker part. This enables a comparison of the additional readout with the readout and, in particular, determine any changes in the optical properties of the labelling moieties. In particular, the readout is generated only subsequently after the biological sample is incubated in the presence of both marker parts and the binding molecule. The additional readout and the readout may be compared to determine if target analyte is present or if two respective target analytes are in proximity.

The method for detecting target analytes of a biological sample and the marker have the same advantages as the label. Further, the method and marker may be supplemented with the features of the label described in this document, in particular, the features of the dependent claims of the label.

### Short Description of the Figures

Hereinafter, specific embodiments are described referring to the drawings, wherein:
- Fig. 1: is a schematic view of a first label and an affinity reagent,
- Fig. 2: is a schematic view of a marker comprising a first marker part and a second marker part,
- Fig. 3: is a schematic view of the first marker part and a target analyte,
- Fig. 4: is a schematic view of the first marker part, the second marker part and the target analyte, and
- Fig. 5: is a workflow of a method for detecting analytes.

### Detailed Description

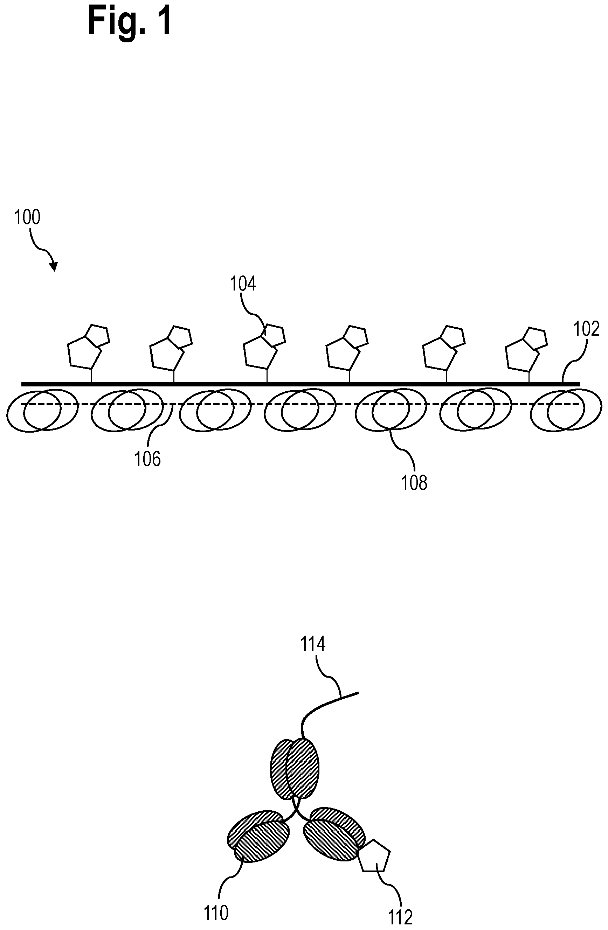

Figure 1 is a schematic view of a label 100 for analysing a biological sample (not shown). The label 100 comprises a first nucleic acid strand 102, to which several labelling moieties 104 are attached. The labelling moieties 104 are preferably covalently attached to the nucleic acid strand 102. The label 100 may have at least one labelling moiety 104. In the case of the label 100 shown in Fig. 1 there are six labelling moieties 104 attached to the nucleic acid strand 102 as an example. The label further comprises a second nucleic acid strand 106 configured to form a duplex structure with the first nucleic acid strand 102.

For example, the first and second nucleic acid strands 102, 106 may be at least partially complementary to each other, with the duplex structure formed by the complementary parts of the first and second nucleic acid strands 102, 106. Thus, the duplex structure may be a double stranded region, which may form a double helix. The first and second nucleic acid strands 102, 106 may form several duplex structures. For example, this may be the case when the first and second nucleic acid strands 102, 106 have several complementary sequence stretches that are separated from each other along the first and second nucleic acid strands 102, 106.

The label 100 further comprises at least one binding molecule 108. The binding molecule 108 is configured to bind to the duplex structure of the first and second nucleic acids strands 102, 106. In case of the label 100, the first and second nucleic acid strands 102, 106 are, in particular, entirely complementary to each other and may form the duplex structure along their entire length. Thus, the binding molecules 108 may bind to the duplex structure of the first and second nucleic acid strands 102, 106 along their entire length.

The labelling moieties 104 may be fluorophores, for example. Thus, the labelling moieties may be optically detectable, for example, based on their fluorescent excitation and/or emission wavelength, and/or fluorescent lifetime. The labelling moieties 104 of label 100 all have the same optical properties, such as excitation and emission wavelength and emission lifetime.

Alternatively, the label 100 may have labelling moieties 104 with different optical properties. In case the combination of the optical properties of the labelling moieties 104 of the label 100 is unique within a set of different labels, the label 100 can be identified based on the unique combination of optical properties of its labelling moieties 104.

In particular, the binding molecules 108 are small molecules that bind to the duplex structure of the first and second nucleic acid strands 102, 106. Binding of the binding molecules 108 to the duplex structure may be specific to a particular sequence of the first and second nucleic acid strands 102, 106 or it may be unspecific to the sequence. For example, the binding molecules 108 may intercalate with the duplex structure or bind to a groove of the duplex structure. Thus, the binding of the binding molecules 108 is based on intermolecular forces such as hydrogen bonding, dipole-dipole interactions, and van der Waals forces.

In particular, the binding molecules 108 are configured to increase the persistence length of the duplex structure of the first and second nucleic acid strands 102, 106. Thus, when the binding molecules 108 are bound to the duplex structure, the duplex structure increases in persistence length or rigidity. This is in addition to an increase in persistence length of the first and second nucleic acid strands 102, 106 due to the formation of the duplex structure. Thus, the label 100 provides a rigid structure for the labelling moieties 104.

Thus, the label 100 enables bright emission of the plurality of labelling moieties 104, in particular with native optical properties of the labelling moieties 104, at a high efficiency. The duplex structure of the first and second nucleic acid strands 102, 106 provides a rigid or stiff backbone to attach the labelling moieties 104 to. The rigidity or stiffness enables keeping the labelling moieties 104 in place relative to each other and avoiding an aggregation of the labelling moieties 104, for example due to an attractive force between labelling moieties 104. Aggregation of labelling moieties 104 may cause a change of their native optical properties such as their emission intensity and/or absorbance spectra. This makes unambiguous identification of the labelling moieties difficult or impossible. In addition, this aggregation of labelling moieties 104 may cause self-quenching of the labelling moieties 104, which may undesirably reduce the brightness of the label 100.

The binding molecules 108 may be optically detectable, for example, the binding molecules 108 may comprise or may be fluorescent molecules. Alternatively, the binding molecules 108 may not be optically detectable, in particular, the binding molecules 108 may not be fluorescent.

Examples of optically detectable binding molecules 108 include ethidium bromide, SYBR Green, SYBR Safe, propidium iodide, DAPI, 7-AAD (7-aminoactinomycin D), Gel Red, Hoechst 33258, Hoechst 33342, and Hoechst 34580.

Examples of optically undetectable binding molecules 108 include Actinomycin D, Daunorubicin, Doxorubicin, Mitoxantrone, and Proflavine.

The binding molecules 108 may all be chemically identical or they may be different.

In a particular embodiment, the binding molecules 108 may excitonically interact with the labelling moieties 104. In particular, the binding molecules 108 and the labelling moieties 104 may be configured for a nonradiative energy transfer between them. For example, the binding molecules 108 and the labelling moieties 104 may form FRET pairs, in which the binding molecules 108 act as FRET donors and the labelling moieties 104 act as FRET acceptors or vice versa. This may increase the brightness of the label 100, in particular the labelling moieties 104.

The binding molecules 108 may be configured to transiently or permanently bind to the duplex structure. For example, binding molecules 108 may be chosen that have a low affinity for the duplex structure and that have a high dissociation rate from the duplex structure, in particular when incubating a biological sample with the label 100. Alternatively, the binding molecules 108 may be chosen to have a high affinity for the duplex structure and that have a low dissociation rate from the duplex structure, in particular when incubating a biological sample with the label 100.

When the binding molecules 108 only transiently bind to the duplex structure, the persistence length of the label 100 may change over time, depending on whether the binding molecules 108 are bound to the duplex structure or not. Correspondingly, the labelling moieties 104 may change their optical properties when they aggregate due to a low persistence length and disaggregate when the persistence length increases when the binding molecules 108 bind to the duplex structure. This may enable a blinking effect of the labelling moieties 104 in the sense that their detectable optical properties change over time, which may be recorded by a time series of images of the biological sample acquired by a microscope.

In contrast, binding molecules 108 that permanently bind to the duplex structure may not exhibit this effect on the label 100. Instead, the detectable optical properties of respective labelling moieties 104 do not change.

The label 100 may optionally be attached to an affinity reagent 110, such as an antibody. The affinity reagent 110 is configured to specifically bind to a target analyte 112 of the biological sample, for example a target protein of interest.

In particular, the affinity reagent 110 may comprise a barcode oligonucleotide 114, which is at least partially complementary to a part of the first or second nucleic acid strands 102, 106. Alternatively, the affinity reagent 110 may be covalently attached to the label 100.

When the label 100 is attached to the affinity reagent 110 a marker comprising the label 100 and the affinity reagent 110 is generated. The marker may be used to analyse a biological sample. For example, the marker may be used to identify and/or localise the target analyte 112 in the biological sample.

A set of labels may comprise a plurality of the first label 100, for example. The plurality of labels 100 of the set of labels may each have a combination of the labelling moieties 104 with different optical properties. Thus, each label 100 of the set of labels may have a unique combination of labelling moieties 104 based on their optical properties. This enables distinguishing the labels 100 of the set of labels based on their optical properties, in particular the optical properties of their respective combination of labelling moieties 104.

Similarly, a set of markers may comprise a plurality of markers with each marker comprising one of the labels 100 and the affinity reagent 110. In particular, each marker may comprise an affinity reagent 110 configured to specifically bind to a different target analyte 112 of a biological sample. Thus, with the set of markers a biological sample with a plurality of different target analytes may be analysed. In particular, the presence and/or location of the plurality of different target analytes may be determined in the biological sample by means of the set of markers.

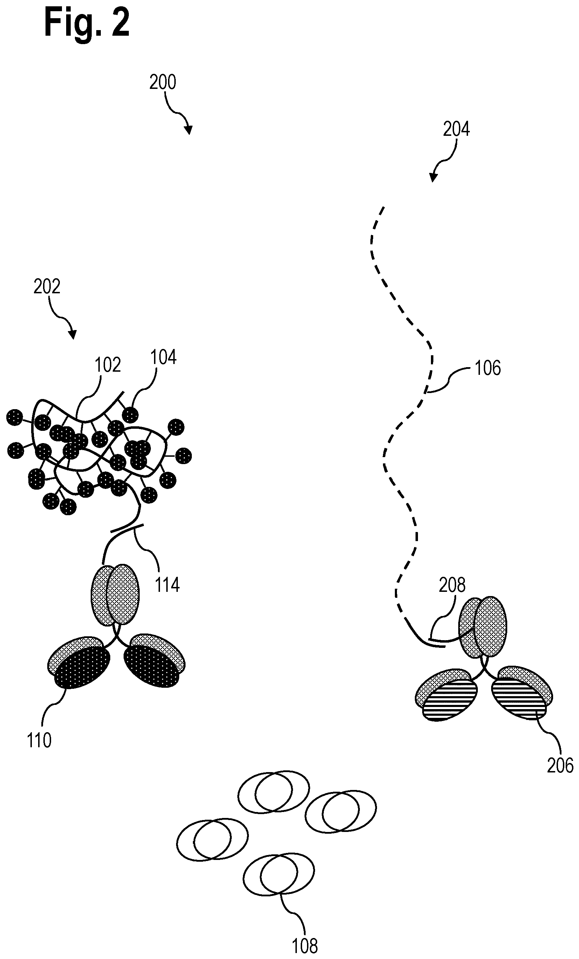

Figures 2 to 4 are schematic views of a marker 200 comprising a first marker part 202 and a second marker part 204.

In the schematic view of Figure 2, individual components of the marker 200 are shown. In particular the first marker part 202 of the marker 200 comprises the first nucleic acid strand 102, to which a plurality of labelling moieties 104 are attached. The first marker part 202 further comprises the (first) affinity reagent 110, which is attached to the first nucleic acid strand 102 via the barcode oligonucleotide 114.

The second marker part 204 comprises the second nucleic acid strand 106 and a second affinity reagent 206. Similarly to the first affinity reagent 110, the second affinity reagent 206 may be an antibody, an antibody fragment, or an aptamer, in particular a nucleic acid based aptamer, for example. The second nucleic acid strand 106 may be attached to the second affinity reagent 206 via a barcode oligonucleotide 208 of the second affinity reagent 206.

Additionally, the marker 200 comprises the binding molecules 108.

In particular in case of the marker 200, the labelling moieties 104 may tend to aggregate. For example, the labelling moieties 104 may be hydrophobic, such that they aggregate in an aqueous solution or under physiological conditions. In Fig. 2 the labelling moieties 104 are shown in an aggregated state. Since the first and second nucleic acid strands 102, 106 are not hybridised, the single stranded first nucleic acid strand 102 has a low persistence length and is flexible to allow the aggregation of the labelling moieties 104.

In their aggregated state, the labelling moieties 104 do not fluoresce with their native optical properties. Rather, the aggregation of the labelling moieties 104 may cause a change in their fluorescence emission due to self-quenching.

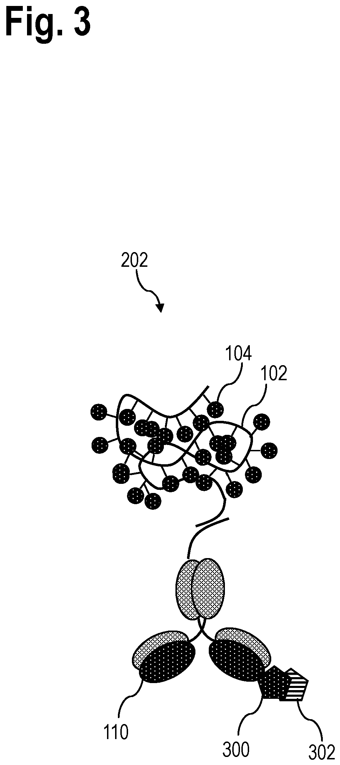

Figure 3 is a schematic view of the first marker part 202 and a first target analyte 300 of a biological sample. The affinity reagent 110 of the first marker part 202 may be configured to specifically bind to the first target analyte 300, which may be a protein, for example. For example, the first marker part 202 may have been introduced into the biological sample (not shown) comprising the target analyte 300 and the first marker part 202, in particular the affinity reagent 110, may have subsequently bound to the target analyte 300. The first target analyte 300 may bind to a second target analyte 302.

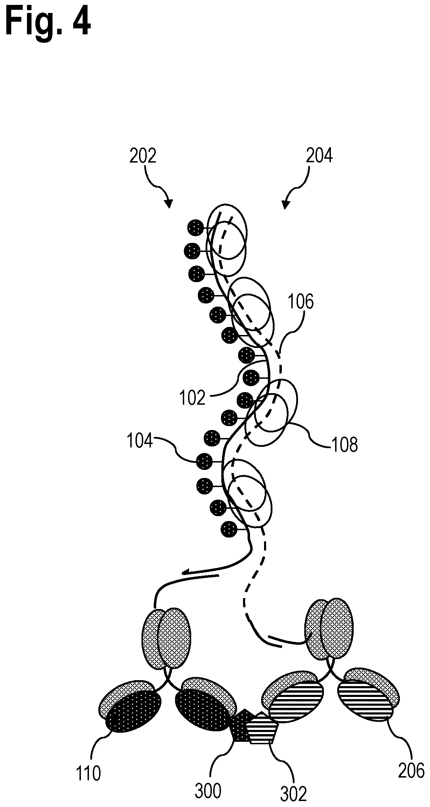

Figure 4 is a schematic view of the first marker part 202, the second marker part 204, and the first and second target analytes 300, 302. The second affinity reagent 206 of the second marker part 204 may be configured to specifically bind to the second target analyte 302. Compared to Fig. 3, the second marker part 204 and the binding molecules 108 have been added to the first marker part 202 in Fig. 4. The addition of the second marker part 204 results in binding of the second marker part 204 to the second target analyte 302. Due to the first and second target analytes 300, 302 being bound to each other, e.g. they are in close proximity, the first and second marker parts 202, 204 are in close proximity. Thus, the first and second nucleic acid strands 102, 106 may hybridise and form the duplex structure. In addition, the binding molecules 108 may bind to the formed duplex structure. This increases the persistence length of the first nucleic acid strand 102 and disaggregates the labelling moieties 104 attached to the first nucleic acid strand 102.

The disaggregated labelling moieties 104 may fluoresce at their native optical properties, which may be detected in an optical readout of the marker 200 and the target analytes 300, 302. The difference in optical properties between the aggregated and disaggregated labelling moieties 104 may be determined and based on this the close proximity and interaction of the target analytes 300, 302 may be determined. Thus, by means of the marker 200, the interaction between the first and second target analytes 300, 302 may be determined.

In particular, the self-quenching of the labelling moieties 104 in their aggregated state may result in a reduced fluorescence intensity of the labelling moieties 104 in the aggregated state. In comparison, the disaggregated labelling moieties 104, in particular, when the duplex is formed and the binding molecules 108 are bound to the duplex, enables an increased fluorescence intensity of the labelling moieties 104. Based on the increase in fluorescence intensity, the close proximity of the target analytes 300, 302 may be determined.

In particular, the labelling moieties 104 may be arranged on the first nucleic acid strand 102 at a distance from each other, at which the labelling moieties 104 do not self-quench, in particular, when the duplex structure is formed between the first and second nucleic acid strands 102, 106.

In particular, the binding molecules 108 may act as FRET donors to the labelling moieties 108. In this case, the addition of the binding molecules 108 may further increase the fluorescence intensity of the labelling moieties 104 or enable a FRET detection of the marker 200. Since the binding molecules 108 only bind to the duplex structure, they only increase the fluorescence intensity, if the marker parts 202, 204 are in close proximity, in particular, due to being bound to the target analytes 300, 302.

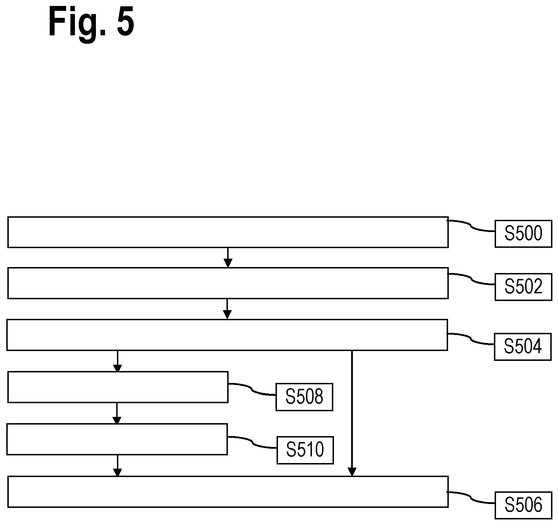

Figure 5 is a workflow of a method for detecting target analytes in a biological sample. The method starts in step S500. In step S502, the biological sample is incubated in the presence of a marker, such as the marker generated from the label 100 and the affinity reagent 110. In particular, the biological sample is incubated such that the marker may bind to the target analyte in the biological sample. In particular, the affinity reagent 110 is configured to specifically bind to the target analyte of the biological sample. In a subsequent step S504, a readout is generated of the marker and the biological sample. The readout may be an optical readout, in particular. For example, the readout may be generated by means of a microscope.

The labelling moieties 104 of the label 100 of the marker may be determined in the readout and the presence and/or location of the target analyte may be determined in the biological sample.

The method ends in steps S506.

In an alternative embodiment of the method, the marker 200 may be used in the method. In this case, only the first marker part 202 may be incubated with the biological sample in step S502. Fig. 3 may be an exemplary view of the first marker part 202 bound to the target analyte 300 in the biological sample.

In step S504, the readout is generated of the first marker part 202 and the biological sample. In step S508, the second marker part 204 is subsequently incubated with the biological sample, which already comprises the first marker part 202. In particular, in step S508 the biological sample is incubated with the first and second marker parts 202, 204 such that the second marker part 204 can bind to the second target analyte 302 and hybridise to the first marker part 202.

In step S510 an additional readout is generated of the biological sample with the first and second marker parts 202, 204. The close proximity of the first and second target analytes 300, 302 may be determined based on the optical properties of the labelling moieties 104 determined in the readouts generated in steps S504 and S510.

Alternatively, the set of markers may be incubated with the biological sample in step S502. In particular, the set of markers may be based on the set of labels, as described above. This enables determining the presence and/or location of a large number of target analytes in the biological sample.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### List of Reference Signs

- 100: Label
- 102: First nucleic acid strand
- 104: Labelling moiety
- 106: Second nucleic acid strand
- 108: Binding molecule
- 110, 206: Affinity reagent
- 112, 300, 302: Target analyte
- 114, 208: Barcode oligonucleotide
- 200: Marker
- 202: First marker part
- 204: Second marker part

## Claims

1. A label (100) for analysing a biological sample comprising:
a first nucleic acid strand (102) and a second nucleic acid strand (106), wherein the first nucleic acid strand (102) and the second nucleic acid strand (106) are configured to form at least one duplex structure,
a first plurality of optically detectable labelling moieties (104) attached to the first nucleic acid strand (102), and
at least one binding molecule (108) configured to bind to the at least one duplex structure.

2. The label according to claim 1, wherein the first nucleic acid strand (102) and the second nucleic acid strand (106) are at least partially complementary.

3. The label according to one of the preceding claims, wherein the binding molecule (108) is configured to intercalate with the at least one duplex structure or to bind to a groove of the at least one duplex structure.

4. The label according to one of the preceding claims, wherein the binding molecule (108) is configured to bind to the duplex structure transiently or permanently.

5. The label according to one of the preceding claims comprising a plurality of the at least one binding molecule (108).

6. The label according to one of the preceding claims, wherein the binding molecule (108) is optically detectable.

7. The label according to one of the preceding claims, wherein the plurality of labelling moieties (104) and the at least one binding molecule (108) are configured to excitonically interact with each other.

8. The label according to one of the preceding claims comprising at least 5 of the labelling moieties (104).

9. The label according to one of the preceding claims, wherein the plurality of labelling moieties (104) are attached to the at least one duplex structure.

10. The label according to one of the preceding claims, wherein the plurality of labelling moieties (104) are hydrophobic labelling moieties.

11. A marker (200) for analysing a biological sample with a plurality of target analytes comprising:
a label (100) according to one of the preceding claims, and
a first affinity reagent (110) configured to specifically bind to one of the target analytes (112, 300).

12. The marker according to claim 11 comprising a first marker part (202) comprising the first nucleic acid strand (102) of the label (100) and the first affinity reagent (110), and a second marker part (204) comprising the second nucleic acid strand (106) of the label (100) and a second affinity reagent (206) configured to specifically bind to one of the target analytes (302).

13. A method for detecting target analytes of a biological sample, the method comprising the following steps:
incubating the biological sample in the presence of at least one marker (200) according to one of the preceding claims 11 and 12,
acquiring a readout of the at least one marker (200) and the biological sample.

14. The method according to claim 13, wherein in the step of incubating the biological sample in the presence of the at least one marker (200), in particular the marker according to claim 12, initially, the biological sample is incubated in the presence of the first marker part (202), and subsequently the second marker part (204) and the at least one binding molecule (108) is added to the biological sample.

15. The method according to claim 14, wherein an additional readout is acquired after incubating the biological sample in the presence of the first marker part (202).
